# EUROPÄISCHE PATENTSCHRIFT

(11) **EP 1 401 333 B1**
(45) Veröffentlichungstag und Bekanntmachung des Hinweises auf die Patenterteilung: **14.02.2007**
(21) Anmeldenummer: 02758228.7
(22) Anmeldetag: 12.06.2002
(51) Int. Cl.: A61B 5/15

(54) **BLUTENTNAHMESPRITZE**
BLOOD SAMPLE SYRINGE
SERINGUE DE PRELEVEMENT DE SANG

(30) Priorität: 30.06.2001 DE 20110872 U
(43) Veröffentlichungstag der Anmeldung: 31.03.2004
(73) Patentinhaber: Büttner, Klaus, D-25336 Klein Nordende (DE)
(72) Erfinder: Büttner, Klaus, D-25336 Klein Nordende (DE)
(74) Vertreter: Glaeser, Joachim
(86) Internationale Anmeldenummer: PCT/EP2002/006413
(87) Internationale Veröffentlichungsnummer: WO 2003/003922

(56) Entgegenhaltungen:
- EP-A- 0 847 726
- WO-A-01/03756
- US-A- 3 645 253
- US-A- 5 725 832

## Beschreibung

Die Erfindung bezieht sich auf eine Blutentnahmespritze, aufweisend einen über eine Hohlnadel zugänglichen zylindrischen Blutaufnahmeraum mit einem darin bewegbaren Kolben mit einem sich nach außen erstreckenden Stempel.

Dokument US 3 645 253 A offenbart eine Blutentnahmespritze wobei der Stempel benachbart zum Kolben mit einer Sollbruchstelle versehen ist.

Für die autologe Transfusion oder Bluttransfusion von Eigenblut bzw. aufbereiteten Eigenblutbestandteilen wird das dem Patienten vor einem geplanten operativen Eingriff abgenommene Blut zentrifugiert und auf Spritzen entzogen, so dass in mehr oder weniger großen zeitlichen Abständen nach dem operativen Eingriff dem Patienten, vor allem zur Kompensation größerer Blutverluste, Eigenblut zugeführt werden kann. Die Vorteile dieses Verfahrens sind darin zu sehen, dass es zu keinen immunitätsbedingten Transfusionszwischenfällen kommen kann, bzw. dass keine Übertragung von fremden Krankheitserregern möglich ist. Allerdings ist es von der Blutentnahme ab bis zur Retransfusion des Blutserums erforderlich, besondere Reinheitsbedingungen strikt einzuhalten.

Bei bekannten Verfahren wird die Blutentnahmespritze eingesetzt, um dem Patienten Blut zu entnehmen, und die Spritze wird in einem Kühlbehälter in ein Labor transportiert. Dort wird das Blut der Entnahmespritze entnommen und in Zentrifugenröhrchen überführt. Die Zentrifugenröhrchen werden in eine Zentrifuge eingesetzt und nach der Zentrifugation kann aus dem oberen Bereich der Zentrifugenröhrchen mit Hilfe einer Serumentnahmenadel Serum entnommen werden, wobei korpuskuläre Bestandteile im unteren Bereich der Zentrifugenröhrchen verbleiben. Bei der Entnahme des Serums müssen die Zentrifugenröhrchen geöffnet werden, so dass insgesamt zwei Vorgänge den höchsten klinischen Ansprüchen entgegenstehen, nämlich das Einfüllen des Blutes in die Zentrifugenröhrchen und die Entnahme des Serums.

Hier nun setzt die vorliegende Erfindung an und will eine Blutentnahmespritze der eingangs genannten Art so ausgestalten, dass für das gesamte Verfahren von der Blutentnahme bis zur Überführung des Serums in die geeigneten Serumspritzen die Bedingungen eines geschlossenen Systems gelten.

Erreicht wird dies insbesondere dadurch, dass bei einer Blutentnahmespritze der eingangs genannten Art der Stempel mit einer Sollbruchstelle ausgebildet wird, um den Stempel in einen abnehmbaren Teil und einen mit dem Kolben fest verbundenen Teil zu zerlegen, wobei der Kolben mit einer Durchstichstelle für die Serumentnahmenadel ausgebildet ist, so dass das entnommene Blut während des Zentrifugierens bis zur Entnahme des Blutserums nicht der Spritze entnommen werden muss.

Beim Einsatz der Blutentnahmespritze gemäß der Erfindung verbleibt das entnommene Blut in der Blutentnahmespritze, und zwar bis zu dem Zeitpunkt, wo das separierte Serum mit Hilfe einer Serumentnahmenadel entnommen werden kann. Umfüllvorgänge, die bei bekannten Blutentnahmespritzen erforderlich sind, können entfallen.

Um nun nicht die Zentrifugen übermäßig groß auszugestalten, wird bei der Blutentnahmespritze gemäß der Erfindung der Stempel zweiteilig ausgebildet oder in zwei Teile zerlegbar ausgebildet, so dass nach der Aufnahme des Blutes, also dem Ausfahren des Stempels die Längserstreckung, gekürzt wird. Auf diese Art und Weise verringert sich auch die radiale Erstreckung der Blutentnahmespritze in der Zentrifuge, was bedeutet, dass bislang eingesetzte Zentrifugen auch im Zusammenhang mit der Blutentnahmespritze gemäß der Erfindung weiterhin eingesetzt werden können.

Nachdem der Zentrifugiervorgang vollendet worden ist, kann mit Hilfe der Serumentnahmenadel in den Innenraum der Blutentnahmespritze eingedrungen werden, indem entsprechende Wandungen im Kolben oder eine dort vorgesehene Membran durchstochen wird. Der Zugang für die Serumentnahmenadel wird dadurch freigelegt, dass die beiden Stempelteile an der Sollbruchstelle voneinander getrennt werden.

Insgesamt wird durch die Blutentnahmespritze gemäß der Erfindung ein geschlossenes System realisiert, was bislang nicht der Fall war.

Die beiden Teile des Stempels können zusammen mit dem Kolben in einem Stück aus Kunststoff gespritzt werden. Denkbar ist auch, dass die beiden Stempelteile durch irgendeine Verbindung zusammengefügt werden, die lösbar ist, bevor die Blutentnahmespritze in die Zentrifuge eingesetzt wird.

Weitere vorteilhafte Ausführungsformen gehen aus den Merkmalen der Unteransprüche hervor.

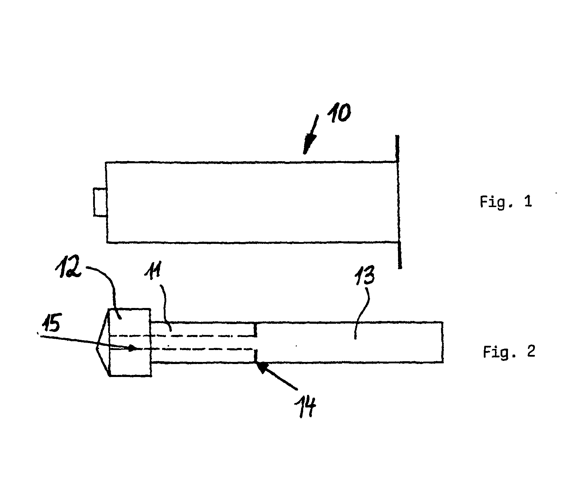

Fig. 1 und 2 zeigen eine Entnahmespritze gemäß der Erfindung, und zwar den eigentlichen Zylinder neben dem Stempel.

In der Figur ist mit 10 der Zylinder einer Blutentnahmespritze gezeigt. In diesem Zylinder ist der Kolben 12 hin- und herbewegbar, wobei diese Bewegung über den angespritzten Stempel 11, 13 bewerkstelligt wird. Die beiden Stempelteile 11 und 13 sind an einer Sollbruchstelle 14 zerlegbar, wobei in der gezeigten Ausführungsform die Sollbruchstelle sich näher zur Mitte der Längserstreckung des Kolbens hin orientiert. Die optimale Lage richtet sich eher an der Nachbarschaft zum Kolben aus, weil im gefüllten Zustand des Innenraums des Zylinders nach der Abtrennung des Teiles 13 vom Teil 14 die Blutentnahmespritze die kürzeste Erstreckung einnimmt.

Mit 15 ist ein Kanal gezeigt, der im Bereich der Sollbruchstelle 14 offen ist, sich mittig im Stempel 11 befindet und im vorderen Bereich in der Stirnfläche des Kolbens 12 endet. Dort befindet sich entweder eine relativ dünne Wand des Kolbens oder aber eine gummiartige Membran, so dass durch Einführen der Serumentnahmespritze in den Kanal 15 diese Stelle durchstochen werden kann und das sich im Inneren der Blutentnahmespritze befindliche Blut bzw. das dort befindliche Serum abgezogen werden kann.

Bei der Benutzung wird der Stempel 11, 13 in den Zylinder 10 bei Blickrichtung der Figur von rechts her nach links vollständig eingefahren. Durch eine nach rechts gerichtete Bewegung des Kolbens 12 kann nunmehr Blut in den Innenraum des Zylinders 10 angesaugt werden. Dadurch gelangt der Stempel 13 immer weiter aus dem Innenraum des Zylinders 10 heraus. Nunmehr kann die Sollbruchstelle 14 verwendet werden, um den Stempel 13 vom Stempel 11 abzutrennen. In diesem Zustand wird die Blutentnahmespritze in die Zentrifuge getan und nach Trennung durch die Zentrifugalkräfte kann das Serum aus dem Innenraum des Zylinders 10 dadurch entnommen werden, dass eine Serumentnahmespritze durch den Kanal 15 von rechts her eingeführt wird. Im Bereich der Sollbruchstelle ist eine Öffnung durch Trennen der Stempel 11 und 13 voneinander vorhanden. Am vorderen Ende des Kolbens 12 geht die Spitze der Serumspritze durch die dort vorhandene Membran hindurch und nunmehr kann problemlos das Serum abgezogen werden.

Es ist ersichtlich, dass von der Blutentnahme bis zur Serumentnahme ein geschlossenes System vorhanden ist.

## Patentansprüche

1. Blutentnahmespritze, aufweisend einen über eine Hohlnadel zugänglichen zylindrischen Blutaufnahmeraum mit einem darin bewegbaren Kolben mit einem sich nach außen erstreckenden Stempel, wobei der Stempel (11, 13) benachbart zum Kolben (12) mit einer Sollbruchstelle (14) oder einer solchen lösbaren Verbindung von zwei Stempelteilen (11, 13) ausgebildet ist, um den Stempel zu zerlegen in einen abnehmbaren Teil (13) und einen mit dem Kolben (12) fest verbundenen Teil (11), **dadurch gekennzeichnet, dass** der Kolben (12) mit einer Membran oder einer Durchstichstelle für eine Serumentnahmenadel ausgebildet ist, und dass an der Sollbruchstelle (14) ein Zugangskanal (15) für die Serumentnahmenadel ausgebildet ist, der sich zur Durchstichstelle erstreckt.

2. Blutentnahmespritze nach Anspruch 1, **dadurch gekennzeichnet, dass** der Kolben (12) und der Stempel (11, 13) einstückig aus Kunststoff gespritzt sind.

3. Blutentnahmespritze nach einem der Ansprüche 1 oder 2, **dadurch gekennzeichnet, dass** die Durchstichstelle und der Zugangskanal (15) zentral zur Längserstreckung der Spritze ausgebildet sind.

4. Blutentnahmespritze nach einem der Ansprüche 1 bis 3, **dadurch gekennzeichnet, dass** die Serumentnahmenadel an einem Verteilerstück angebracht ist, von dem aus das entnommene Serum in eine Mehrzahl von Spritzen gegeben wird.

## Claims

1. Blood sample syringe comprising a cylindrical blood receiving space accessible through a hollow needle, said space having a piston moveable therein with a plunger extending outwardly, wherein said plunger (11, 13) is formed with a predetermined breaking point (14) adjacent to the piston (12) or such a detachable connection of two plunger parts (11, 13), in order to separate the plunger into a detachable part (13) and a part fixedly connected with the piston (12), **characterised in that** the piston (12) is formed with a membrane or a puncturing point for a serum sample needle, and that an access channel (15) for the serum sample needle is formed at the predetermined breaking point (14), which extends to the puncturing point.

2. Blood sample syringe according to claim 1, **characterised in that** the piston (12) and the plunger (11, 13) are integrally formed of plastic by injection moulding.

3. Blood sample syringe according to one of the claims 1 or 2, **characterised in that** the puncturing point and the access channel (15) are formed centrally with regard to the longitudinal extension of the syringe.

4. Blood sample syringe according to one of the claims 1 to 3, **characterised in that** the serum sample needle is attached to a distributor, from which the sampled serum is discharged into a plurality of syringes.

## Revendications

1. Seringue de prélèvement de sang comportant une chambre cylindrique de réception du sang qui est accessible par une aiguille creuse et dans laquelle se déplace un piston avec un poinçon se prolongeant vers l'extérieur, ledit poinçon (11), (13) qui est adjacent du piston (12) comportant une zone de rupture (14) prédéterminée ou une liaison détachable entre les deux parties (11), (13) du poinçon, de sorte que le poinçon soit composé d'une partie amovible (13) et d'une partie (11) solidaire du piston (12), **caractérisée en ce que** le piston (12) comporte une membrane ou un point de percement destiné à l'aiguille de prélèvement de sérum et que un canal d'accès (15) destiné à l'aiguille de prélèvement de sérum et s'étendant jusqu'au point de percement est prévu dans la zone de rupture prédéterminée (14).

2. Seringue selon la revendication 1 **caractérisée en ce que** le piston (12) et le poinçon (11), (13) forment une pièce unique extrudée en matière synthétique.

3. Seringue selon l'une des revendications 1 à 2 **caractérisée en ce que** le point de percement et le canal d'accès (15) sont agencés au centre par rapport à l'étendue longitudinale de la seringue.

4. Seringue selon l'une des revendications 1 à 3 **caractérisée en ce que** l'aiguille de prélèvement de sérum est disposée sur une pièce de répartition à partir de laquelle le sérum prélevé est réparti dans plusieurs seringues.
